# EUROPEAN PATENT APPLICATION

(11) **EP 3 015 076 A1**
(43) Date of publication of application: **04.05.2016**
(21) Application number: 14817543.3
(22) Date of filing: 19.06.2014
(51) Int. Cl.: A61B 17/00

(54) **APPLICATOR**

(30) Priority: 26.06.2013 JP 2013134120; 26.03.2014 JP 2014063290
(71) Applicant: Anest Iwata Corporation, Yokohama-shi, Kanagawa 223-8501 (JP); 3-D Matrix Ltd., Chiyoda-ku Tokyo 102-0083 (JP)
(72) Inventor: ISHIKAWA, Hidetoshi, Yokohama-shi Kanagawa 223-8501 (JP); KUBO, Masahiro, Yokohama-shi Kanagawa 223-8501 (JP)
(74) Representative: Hoffmann Eitle
(86) International application number: PCT/JP2014/066226
(87) International publication number: WO 2014/208433

(57) **Abstract**

An applicator (10) has a syringe (11) filled with a drug such as a hemostatic or a biological adhesive. A drug ejecting part (12) is provided to one end of the syringe (11). A plunger (13) is inserted from the other end of the syringe (11). A gas flow passage (17) for supplying a compressed gas to the drug ejecting part (12) is formed in a support part (14) for supporting the syringe (11). The gas flow passage (17) has a valve (18). The valve (18) is opened by pressing of a trigger (16) attached to a grip part (15) of the support part (14). An operator grasps the grip part (15) in one hand. When the operator presses a pressing part (13A) of the plunger (13) with a thumb, the drug in the syringe (11) is ejected from a nozzle (12A) of the drug ejecting part (12). When the operator presses the trigger (16) with an index finger, the compressed gas is ejected from an air cap (12B) of the drug ejecting part (12). When the drug and compressed air are simultaneously ejected, the drug is atomized.

## Description

### TECHNICAL FIELD

The present invention relates to an applicator, and relates to an applicator for applying, for example, a hemostatic agent.

### BACKGROUND ART

Known examples of this type of applicator include the following patent literatures, PTLs 1 and 2.

PTL 1 discusses a surgical injector configured to be able to arbitrarily inject pressurized gas, and a hemostatic agent and a procoagulant. This surgical injector includes a means for supplying the pressurized gas, a means for supplying the hemostatic agent, a means for supplying the procoagulant, and an injection nozzle.

PTL 2 discusses a simple applicator for a biocompatible adhesive. This simple applicator for the biocompatible adhesive includes a spray head including a sterilized gas supply flow passage formed therein, a pair of syringe members to which the spray head is fitted, a high-pressure regulator, and a compact high-pressure gas canister, which are prepared integrally with one another. Further, this simple applicator is configured in such a manner that the spray head and the high-pressure regulator are connected to each other via a tube, and sterilized gas is supplied after passing through an air filter. Then, the applicator is configured in such a manner that a fibrinogen solution and a thrombin solution are loaded into the syringe members, respectively, and are mixed together within the spray head and then atomized.

### CITATION LIST

### PATENT LITERATURE

PTL 1: Japanese Patent Application Public Disclosure No. S61-79452
PTL 2: Japanese Utility Model Application Public Disclosure No. H6-52839

### SUMMARY OF INVENTION

### TECHNICAL PROBLEM

The surgical injector discussed in PTL 1 includes the injection nozzle. The hemostatic agent is supplied from a hemostatic agent reserve chamber into this injection nozzle through a tube via a valve provided on the way in this tube. Further, the surgical injector is configured in such a manner that compressed air is supplied from a compressor into this injection nozzle through another tube via another valve provided on the way in this other tube. In this manner, the surgical injector discussed in PTL 1 has disadvantages of a complicated structure thereof, and a lack of user-friendliness in terms of operability for operating these values.

Further, in the simple applicator for the biocompatible adhesive discussed in PTL 2, the fibrinogen solution or the like loaded in the syringe member is supplied into the spray head of the applicator by a pusher. Then, the applicator is configured in such a manner that compressed air is supplied from the high-pressure canister through a tube via a faucet provided on the way in this tube. This applicator has disadvantages of a large size thereof due to the integrated provision of the high-pressure gas canister, the regulator, and the pair of syringe members, and further, a lack of user-friendliness in terms of operability for operating this faucet.

Further, a mainstream of conventional hemostatic agent applicators has been an applicator configured to inject mixed two liquids, the hemostatic agent and the procoagulant. However, the recent advancement of the medical technology has allowed application or spraying of only one liquid to stop bleeding, which has lead to a demand for an applicator configured to inject a single liquid (an applicator including a single syringe).

The present invention has been contrived in consideration of these circumstances, and an object thereof is to provide an applicator having a compact simple structure, extremely excellent operability, a capability to achieve precise application, and improved workability.

### SOLUTION TO PROBLEM

The present invention would be perceivable from the following configurations.
(1) According to an aspect of the present invention, an applicator includes a syringe in which a liquid agent is loaded, a liquid agent injection unit provided at one end of the syringe and arranged into communication with a liquid agent flow passage, a pusher configured to be slidably inserted from an opposite end of the syringe, a push portion provided at an end of the pusher opposite from a syringe side of the pusher that is inserted in the syringe and configured to cause the liquid agent loaded in the syringe to be injected from the liquid agent injection unit by being pushed, a support unit configured to support the syringe, a gas flow passage formed in the support unit and configured to supply compressed gas to the liquid agent injection unit, and an operation unit provided to the support unit and configured to cause a start of the supply of the compressed gas to the liquid agent injection unit. The push portion is located at a position that allows an operator to push the push portion.
(2) According to another aspect of the present application, in the applicator configured as described in the item (1), the push portion may be located at a position that allows the operator to push the push portion with a thumb of a hand engaging the operation unit with at least one finger other than this thumb.
(3) According to another aspect of the present application, in the applicator configured as described in the item (1), the push portion may be located at a position that allows the operator to push the push portion with a thumb while holding the operation unit.
(4) According to another aspect of the present application, in the applicator configured as described in any of the items (1) to (3), the support unit may be configured to be able to support only a single syringe.
(5) According to another aspect of the present application, in the applicator configured as described in any of the items (1), (2), and (4), the operation unit may be provided in such a manner that only the operation unit protrudes from the support unit opposite from the syringe, and the operation unit may be unequipped with a grip unit.
(6) According to another aspect of the present application, the applicator configured as described in the item (5) may further include a gas supply port formed in a vicinity of an end of the support unit where the opposite end of the syringe is located, and extending obliquely so as to be separating from the syringe. The compressed gas may be supplied from a gas supply hose fitted to the gas supply port.
(7) According to another aspect of the present application, the applicator configured as described in any of the items (1) to (4) may further include a grip unit provided to the support unit and including a gas flow passage for supplying the compressed gas to the gas flow passage of the support unit. The push portion may be located at a position that allows the operator to push the push portion while operating the operation unit with a hand holding the grip unit.
(8) According to another aspect of the present application, in the applicator configured as described in the item (7), the grip unit may be disposed offset from the syringe in a direction intersecting with a longitudinal direction of the syringe.
(9) According to another aspect of the present application, in the applicator configured as described in the item (7) or (8), the grip unit may be provided to the support unit via an angle adjustment mechanism.
(10) According to another aspect of the present application, in the applicator configured as described in any of the items (1) to (9), the liquid agent injection unit may include a nozzle, and a gas cap provided around the nozzle on an outer periphery of the nozzle. The gas cap and the nozzle each may be shaped in such a manner that an inner diameter and an outer diameter thereof are reducing toward a distal end thereof.
(11) According to another aspect of the present application, in the applicator configured as described in the item (10), the nozzle may include a rib provided on the outer periphery of the nozzle.
(12) According to another aspect of the present application, in the applicator configured as described in any of the items (1) to (11), the syringe may be supported by the support unit via a lure-lock mechanism.
(13) According to another aspect of the present application, in the applicator configured as described in any of the items (1) to (12), the pusher may be configured to cause the liquid agent to be injected form the liquid agent injection unit by pushing the liquid agent into the syringe.
(14) According to another aspect of the present application, in the applicator configured as described in any of the items (1) to (13), the operation unit may include a mechanism capable of adjusting a supply amount of the compressed gas, and atomize the liquid agent injected form the liquid agent injection unit with use of the compressed gas to spray the atomized liquid agent.
(15) According to another aspect of the present application, in the applicator configured as described in any of the items (1) to (14), the entire applicator may be made from resin.
(16) According to another aspect of the present application, in the applicator configured as described in any of the items (1) to (15), the liquid agent may be any one of a hemostatic agent, an adhesion prevention agent, and a biocompatible adhesive.

### ADVANTAGEOUS EFFECTS OF INVENTION

According to the thus-configured applicator, the operability can be extremely improved despite the simple structure. Further, this configuration allows the applicator to precisely apply the liquid agent to a position to which the liquid agent should be applied, thereby improving the workability.

### BRIEF DESCRIPTION OF DRAWINGS

Fig. 1(A) is a top view of an applicator according to a first embodiment of the present invention.
Fig. 1(B) is a front view of the applicator.
Fig. 1(C) is a back view of the applicator.
Fig. 1(D) illustrates a differently configured applicator, which is used in a composition with Fig. 1(E).
Fig. 1(E) illustrates a position of a thumb when an operator holding the applicator according to the present embodiment, as viewed from a back face.
Fig. 2(A) is a cross-sectional view of a liquid agent injection unit of the applicator.
Fig. 2(B) is a partial enlarged cross-sectional view illustrating a nozzle portion of the applicator in an enlarged manner.
Fig. 3 is a perspective view illustrating a syringe attachment unit provided at a support unit of the applicator.
Fig. 4 illustrates an air flow passage of the applicator, a valve unit provided on the way in this air flow passage, and a trigger for displacing this valve unit.
Fig. 5(A) illustrates a position of the valve unit when the valve unit cuts off communication of the air flow passage.
Fig. 5(B) illustrates a position of the valve unit when the valve unit establishes communication of the air flow passage.
Figs. 6(A) and 6(B) illustrate that a liquid agent in a syringe is injected by a variable injection amount according to how strongly or weakly a pusher is pushed.
Figs. 7(A) and 7(B) each illustrate an applicator according to a second embodiment of the present invention, and illustrate that the grip unit can be inclined with respect to the support unit.
Fig. 8(A) is a plan view of the support unit of the applicator according to the second embodiment.
Fig. 8(B) is an enlarged perspective view illustrating a connection portion of the support unit where the support unit is connected to the grip unit.
Fig. 9 is a perspective view illustrating a configuration of a connection portion of the grip unit where the grip unit is connected to the support unit.
Figs. 10(A) and 10(B) illustrate that a projection formed on the support unit is fitted in any of a plurality of bores formed at the grip unit according to a change in an angle of the grip unit with respect to the support unit.
Fig. 11 illustrates a configuration of an applicator according to a third embodiment of the present invention, and illustrates this applicator in correspondence with Fig. 4.
Fig. 12(A) is a cross-sectional view illustrating an applicator according to a fifth embodiment of the present invention.
Fig. 12(B) is a cross-sectional view illustrating the applicator with the syringe (also including the pusher) removed from the state illustrated in Fig. 12(A).
Fig. 13(A) is a perspective view illustrating a syringe attachment unit included in the applicator according to the fifth embodiment.
Fig. 13(B) illustrates a nozzle from a front face.
Fig. 13(C) is a cross-sectional view illustrating the vicinity of the nozzle.
Fig. 14(A) is a cross-sectional view illustrating the vicinity of a trigger of the applicator according to the fifth embodiment, and illustrates the vicinity of the trigger before the trigger is operated.
Fig. 14(B) illustrates the vicinity of the trigger after the trigger is operated.

### DESCRIPTION OF EMBODIMENTS

In the following description, examples for embodying the present invention (hereinafter referred to embodiments) will be described in detail with reference to the attached drawings. Like elements will be identified by same reference numerals throughout the entire description of the embodiments.

### (First Embodiment)

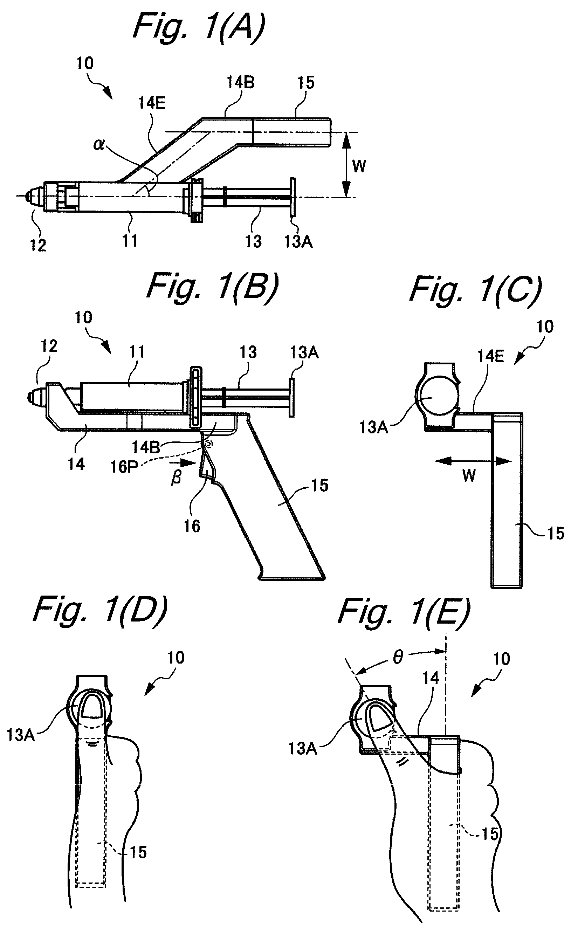

Figs. 1(A), 1(B), and 1(C) illustrate a configuration of a first embodiment of an applicator according to the present invention. Figs. 1(A), 1(B), and 1(C) are a top view, a front view, and a back view, respectively.

An applicator 10 illustrated in Figs. 1(A), 1(B), and 1(C) indicates an applicator for applying, for example, a hemostatic agent (this agent may be referred to as a liquid agent in the present disclosure) containing amino acid. Then, the entire applicator 10 is made from resin.

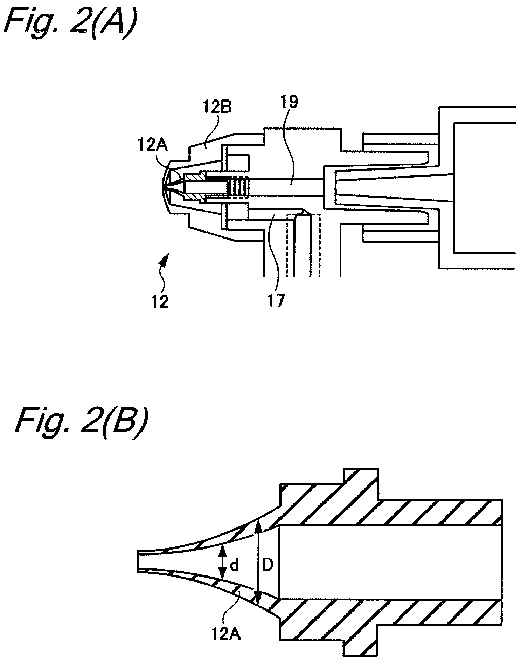

In Figs. 1(A), 1(B), and 1(C), first, the applicator 10 includes a syringe 11, the number of which is, for example, one. A liquid agent injection unit 12 is provided at a front (an end on a left side in the drawings) of the applicator. As illustrated in Fig. 2(A), the liquid agent injection unit 12 includes a nozzle 12A, and an air cap (gas cap) 12B that injects compressed air (compressed gas) for spraying forward the liquid agent injected from a distal end of the nozzle 12A while atomizing the liquid agent. As illustrated in Fig. 2(B), the nozzle 12A is, for example, shaped in such a manner that an inner diameter d and an outer diameter D thereof are reducing toward the distal end thereof. An annular slit defined between the distal end of the nozzle 12A and a central port of the air cap 12B is formed at an atomization head of the liquid agent injection unit 12. In the case of a simple configuration that introduces the compressed air from the annular slit, a spray pattern called a round pattern is formed by the atomization head of the liquid agent injection unit 12. However, the present invention is not limited to this example.

An opening is formed at an opposite end (an end on a right side in the drawings) of the syringe 11. A pusher 13 slidable in this syringe 11 is inserted through this opening.

The applicator 10 is configured to allow the hemostatic agent to be loaded into the syringe 11 with the pusher 13 removed therefrom. The pusher 13 is inserted into the syringe 11, and continues being pushed toward the liquid agent injection unit 12 side while being kept inserted in the syringe 11, by which the hemostatic agent in the syringe 11 is injected from the liquid agent injection unit 12. The applicator 10 may be configured to use a disposable (single-use) type prepared as a cartridge with the liquid agent sealingly contained therein in advance without requiring the hemostatic agent to be loaded later, and the present invention also includes an applicator configured in this manner.

The pusher 13 includes a push portion 13A (refer to Fig. 1(C)) at an end thereof exposed from the syringe 11 (an end on the right side in the drawings). The push portion 13A has a disk-like shape that intersects with an axial direction and is relatively large in diameter. Pushing this push portion 13A allows the pusher 13 to be axially pushed in the syringe 11.

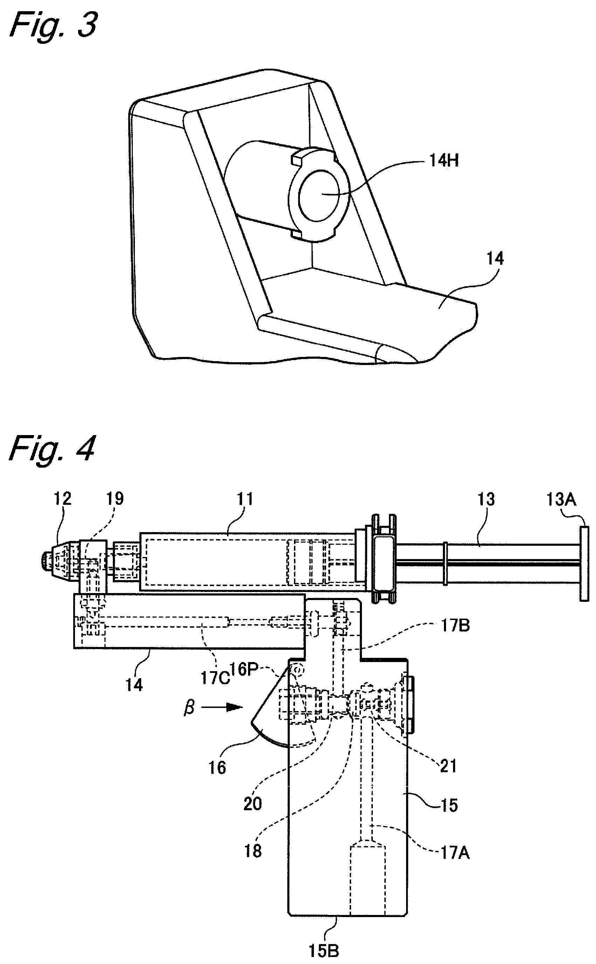

Then, the syringe 11 is configured to be supported by a support unit 14 of the applicator 10. Fig. 3 is a perspective view illustrating a distal end of the support unit 14. A bore 14H, which allows a distal end of the syringe 11 to be inserted therein, is formed at the distal end of this support unit 14.

The distal end of the syringe 11 is inserted in this bore 14H of the support unit 14, by which the liquid agent injection unit 12 is disposed in communication with the syringe 11 via this bore 14H (refer to Fig. 1(B)). The syringe 11 inserted in this bore 14H of the support unit 14 is configured to be quickly and securely fitted to be fixed to the support unit 14 by being rotated by an angle of, for example, approximately 90 degrees. In this manner, the syringe 11 is configured to be supported by the support unit 14 via a so-called lure-lock mechanism.

Now, as illustrated in Fig. 1(A), the support unit 14 includes an extending portion 14E that extends backward at an angle of α (0 degrees < α < 90 degrees) with respect to an axial direction of the syringe 11, and a back portion 14B that extends from the extending portion 14E in parallel with the syringe 11. The back portion 14B of this support unit 14 is formed so as to be horizontally spaced apart from the syringe 11 by a distance W.

Then, a grip unit 15 is provided to the back portion of the support unit 14. The grip unit 15 is provided so as to extend toward an opposite side (a lower side in Figs. 1(B) and 1(C)) of the support unit 14 that is spaced apart from a syringe side where the syringe 11 is located. A trigger 16 (this may be referred to as an operation unit in the present disclosure) is provided at a front of the grip unit 15 at a portion where an operator can easily operate the trigger 16 with his/her index finger while holding the grip unit 15. An upper portion of the trigger 16 is provided to the grip unit 15 via a support axis 16P, and the trigger 16 is configured to be operable by being pushed toward the grip unit 15 side (a direction labeled β in the drawings).

An air flow passage 17 (refer to Fig. 4), through which the compressed air introduced from a bottom of the grip unit 15 is supplied to the liquid agent injection unit 12, is formed in the grip unit 15 and the support unit 14. Operating the trigger 16 causes a start of the supply of this compressed air to the liquid agent injection unit 12.

Fig. 4 is a transparent view illustrating the air flow passage (gas flow passage) 17 formed inside the grip unit 15 and the support unit 14. Fig. 4 illustrates the grip unit 15 and the support unit 14 as if the grip unit 15 is perpendicular to the support unit 14 for convenience sake, but it is apparent that the grip unit 15 may be inclined with respect to the support unit 14 as illustrated in Fig. 1(B).

As illustrated in Fig. 4, the compressed air introduced via an air flow passage 17A extending from a gas supply port at a bottom 15B of the grip unit 15 is guided to an air flow passage 17B via a valve unit 18, and is further guided from the air flow passage 17B to an air flow passage 17C formed in the support unit 14. The air flow passage 17C is connected to the air cap 12B forming the liquid agent injection unit 12 via the air flow passage 17 illustrated in Fig. 2(A). The gas supply port is configured in such a manner that a gas supply hose is fitted thereto.

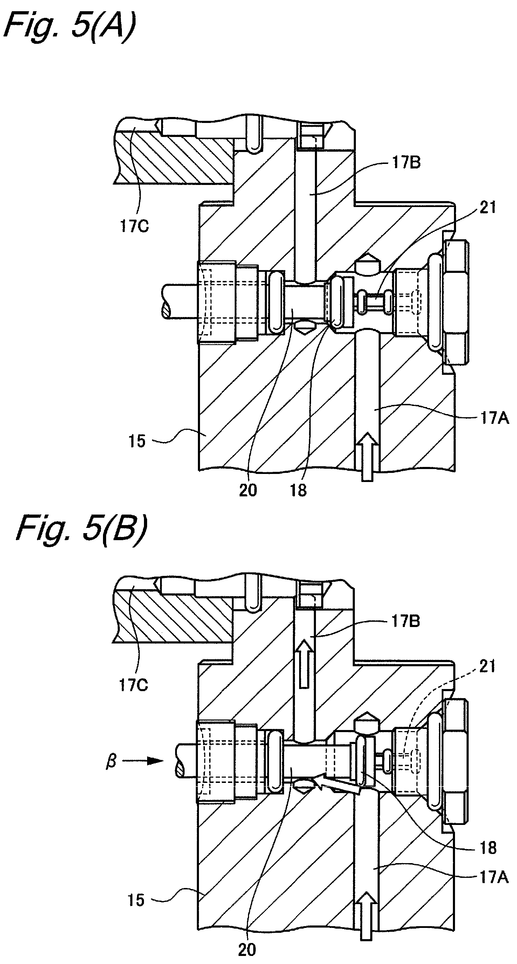

As illustrated in Figs. 5(A) and 5(B), the valve unit 18 is provided on a shaft member 20. The shaft member 20 is biased by a spring 21 toward the trigger 16. Then, a distal end of the shaft member 20 is in abutment with the trigger 16. When the trigger 16 is not pushed in the β direction illustrated in the drawings, as illustrated in Fig. 5(A), the valve unit 18 is seated on a valve seat provided between the air flow passage 17A and the air flow passage 17B, thereby cutting off communication between the air flow passage 17A and the air flow passage 17B. When the trigger is pushed in the β direction illustrated in the drawings, as illustrated in Fig. 5(B), the shaft member 20 is displaced against the biasing force of the spring 21, by which the valve unit 18 is separated from the valve seat, thereby establishing the communication between the air flow passage 17A and the air flow passage 17B.

When the communication is established between the air flow passage 17A and the air flow passage 17B by the operation performed on the trigger 16, the compressed air is injected from the air cap 12B of the liquid agent injection unit 12 via the air flow passage 17A, the air flow passage 17B, and the air flow passage 17C.

The applicator 10 configured in this manner allows the operator to push the push portion 13A of the pusher 13 with his/her thumb and operating the trigger 16 with his/her index finger while holding the grip unit 15 with his/her hand. In this case, the operator can operate the pusher 13 and the trigger 16 independently of each other, and can also operate the pusher 13 and the trigger 16 in combination while adjusting the operations thereof. The operator can also apply the liquid agent while keeping the liquid agent in a liquid state by only operating the pusher 13. Further, the operator can also spray the liquid agent while atomizing the liquid agent by the compressed air by operating both the pusher 13 and the trigger 16. Further, the operator can also inject only the compressed air for the purpose of facilitating drying by operating only the trigger 16.

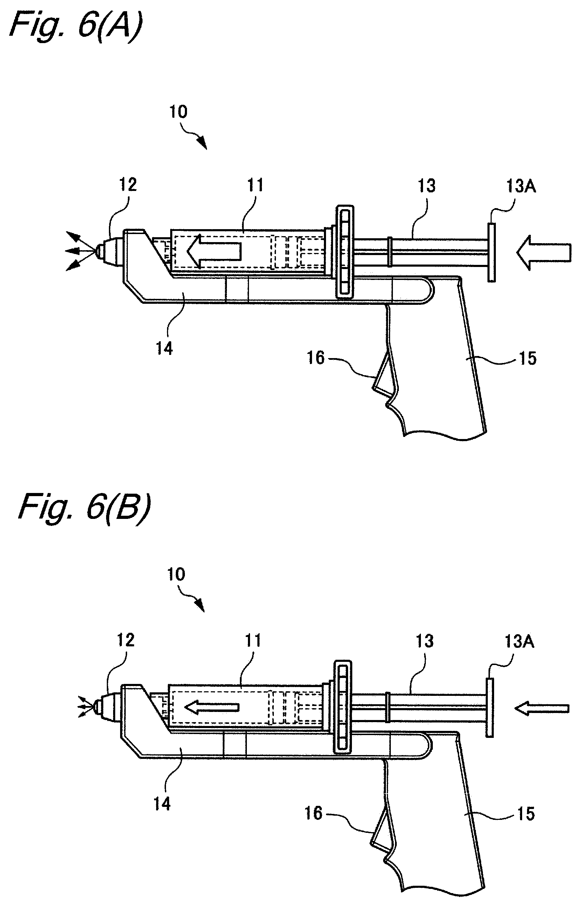

Figs. 6(A) and 6(B) illustrate that the liquid agent can be injected from the liquid agent injection unit by a set injection amount according to strength/weakness of a force pushing the pusher 13. Fig. 6(A) illustrates that an increase in the force (pressure) pushing the pusher 13 (a width of an arrow illustrated in the drawing indicates this increase) causes a strong force to be applied to the liquid agent in the syringe 11, according to which the liquid agent is injected from the liquid agent injection unit 12 by a large amount. Fig. 6(B) illustrates that a reduction in the force (pressure) pushing the pusher 13 (a width of an arrow illustrated in the drawing indicates this reduction) causes a weak force to be applied to the liquid agent in the syringe 11, according to which the liquid agent is injected from the liquid agent injection unit 12 by a small amount.

For example, a configuration using a needle valve can also be used as the configuration for injecting the liquid agent from the liquid agent injection unit 12, but the configuration not using the needle valve can contribute to acquisition of the simply configured applicator.

Similarly, as illustrated in Figs. 5(A) and 5(B), the operation performed on the trigger 16 also allows the compressed air to be injected from the liquid agent injection unit 12 and an injection amount of the compressed air can be set according to strength/weakness of a force pushing the trigger 16, since this operation can change a communication diameter of the air flow passage (the air flow passage 17A and the air flow passage 17B) that is defined by the valve unit 18 according to the displacement of the shaft member 20.

Therefore, the present embodiment provides an advantageous effect of allowing the operator to easily adjust the injection amount of the liquid agent and/or the injection amount of the compressed air that are injected from the liquid agent injection unit 12. Further, the present embodiment allows the operator to simultaneously adjust the respective injection amounts of the liquid agent and/or the compressed air with the thumb of the hand holding the grip unit 15 and the index finger operating the trigger 16, thereby allowing the operator to inject the liquid agent and/or the compressed air in combination while easily adjusting the respective injection amounts thereof.

Further, as illustrated in Fig. 1(C), the grip unit 15 is disposed offset from the syringe 11 in a direction intersecting with a longitudinal direction of this syringe 11, which eliminates any object blocking the operator's view in the vicinity of the syringe 11, bringing about an advantageous effect of allowing the operator to easily take aim when injecting the liquid agent. Further, as illustrated in Fig. 1(E), the first embodiment provides an advantageous effect of preventing the thumb from being easily tired to thereby facilitate pushing the pusher 13, due to separation (an angle θ) that would be naturally generated between fingers when a person holds an object. In the case of Fig. 1(D) in which the grip unit 15 and the syringe 11 are not offset from each other, the operator should raise his/her thumb vertically upward while holding the grip unit 15, which results in inconvenience of significant tiredness of the thumb.

### (Second Embodiment)

The first embodiment has been described as the applicator with the grip unit 15 thereof provided fixedly to the support unit 14.

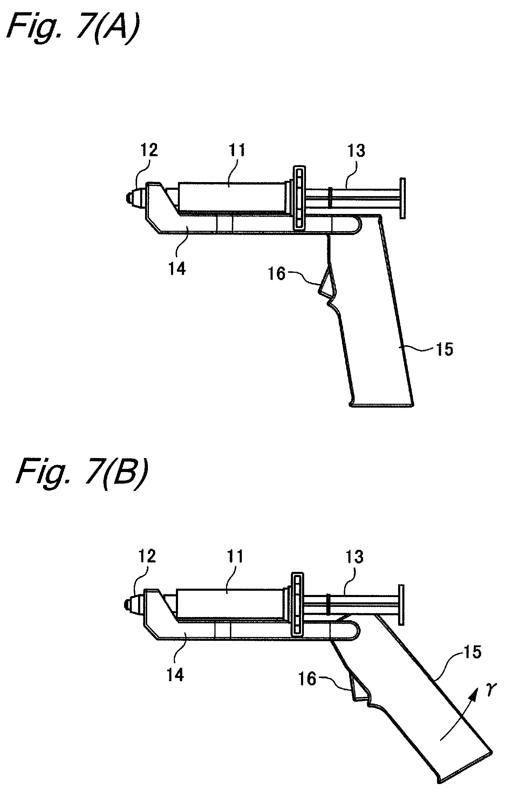

As illustrated in Figs. 7(A) and 7(B), in an applicator according to a second embodiment of the present invention, the grip unit 15 is provided to the support unit 14 via a not-illustrated angle adjustment mechanism, and may be configured to be inclined by a variable degree with respect to this support unit 14 as illustrated by an arrow γ illustrated in the drawing.

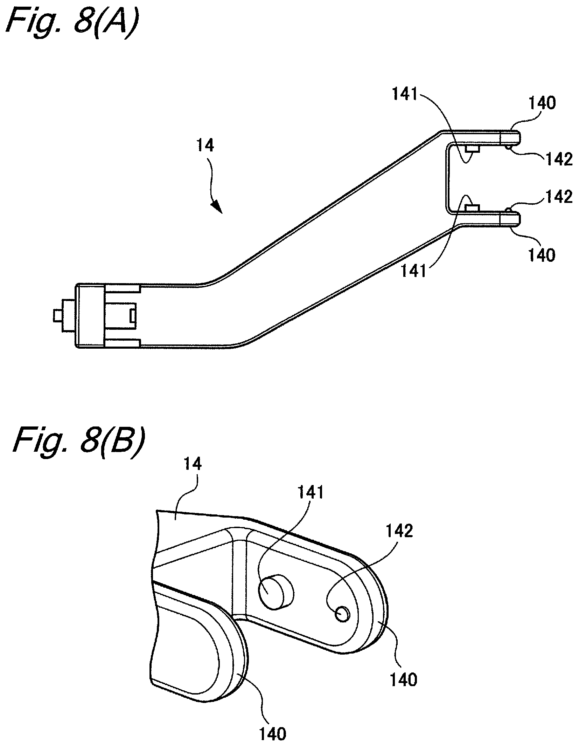

Fig. 8(A) is a plan view illustrating the support unit 14, and Fig. 8(B) is an enlarged perspective view illustrating a connection portion of this support unit 14 where the support unit 14 is connected to the grip unit 15 (not illustrated).

As illustrated in Fig. 8(B), the support unit 14 includes, at one end thereof, the connection portion to which the grip unit 15 (not illustrated) is connected. This connection portion has a fork-like shape including a pair of protruding portions 140. An opposing surface of each of the protruding portions 140 individually includes an axial projection body 141 for pivotally supporting the grip unit 15 (not illustrated), and a projection 142 provided close to this axial projection body 141 for setting an angle.

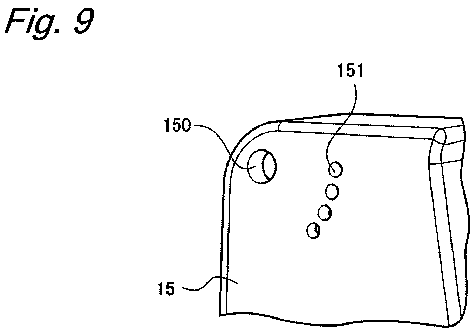

The grip unit 15 (not illustrated in Figs. 8(A) and 8(B)) is disposed between the projection portions 140 of the support unit 14. As illustrated in Fig. 9, the grip unit 15 includes an axial bore 150 into which the axial projection body 141 of the support unit 14 is fitted, and a plurality of bores 151 (for example, four bores 151 in the drawings) into which the projection 142 of the support unit 14 is fitted. The axial bore 150 and the plurality of bores 151 are formed at a portion of the grip unit 15 where the grip unit 15 is connected to the support unit 14 (not illustrated).

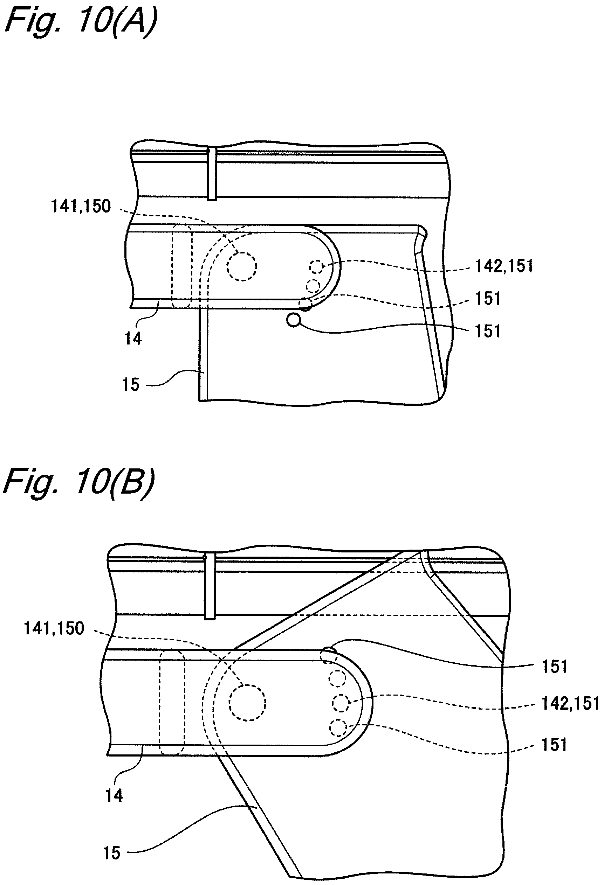

Figs. 10(A) and 10(B) transparently illustrate the support unit 14 with the grip unit 15 provided thereto. The grip unit 15 is configured to be rotatable about the axial projection body 141 (the axial hole 150). In the process of this rotation, the projection 142 is fitted into one selected by the operator among the plurality of bores 151 (the four bores 151 in the drawings). This arrangement allows the operator to adjust the angle of the grip unit 15 through four steps while watching out for a sensation that will be produced when the grip unit 15 is clicked into the support unit 14. An increase or a reduction in the number of bores 151 can expand a range of the angle adjustment.

In this case, as described above, the grip unit 15 is disposed offset from the syringe 11 in the direction intersecting with the longitudinal direction of this syringe 11, which allows the operator to change the inclination of the grip unit 15 in a relatively wide angle range without being obstructed by the syringe 11.

### (Third Embodiment)

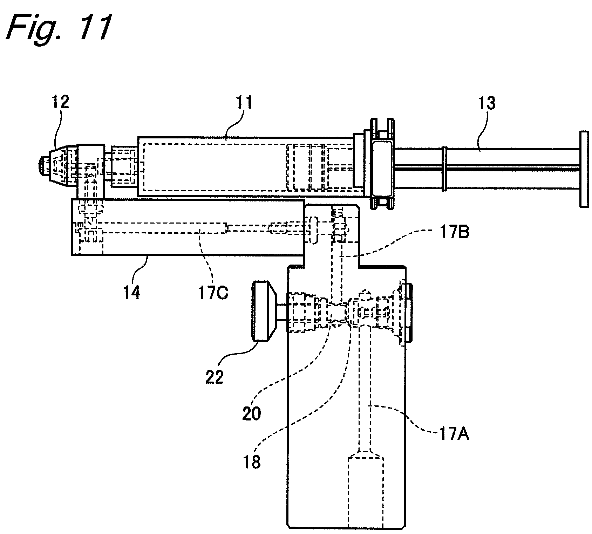

The first embodiment is configured in such a manner that the trigger 16 provided at the grip unit 15 causes the start of the supply of the compressed air to the liquid agent injection unit 12, as illustrated in Fig. 4. However, the start of the supply of the liquid agent is not limited to this example, and the applicator 10 may be configured to include a button 22 at the distal end of the shaft member 20 with the valve unit 18 provided thereon as illustrated in Fig. 11, which illustrates an embodiment in this case in correspondence with Fig. 4. This configuration also allows pressing of the button 22 to cause the start of the supply of the compressed air to the liquid agent injection unit 12, and function as the operation unit capable of adjusting the supply amount of the compressed air by the button 22 being kept pressed for a longer time or a shorter time.

### (Fourth Embodiment)

The above-described embodiments have been described assuming that the liquid agent is the hemostatic agent containing the amino acid for example, by way of example. However, the liquid agent is not limited to this example, and it is apparent that these applicators can be used for applying another liquid agent such as a medical agent, a liquid agent used for the purpose of coating (a coating material), and food liquid.

### (Fifth Embodiment)

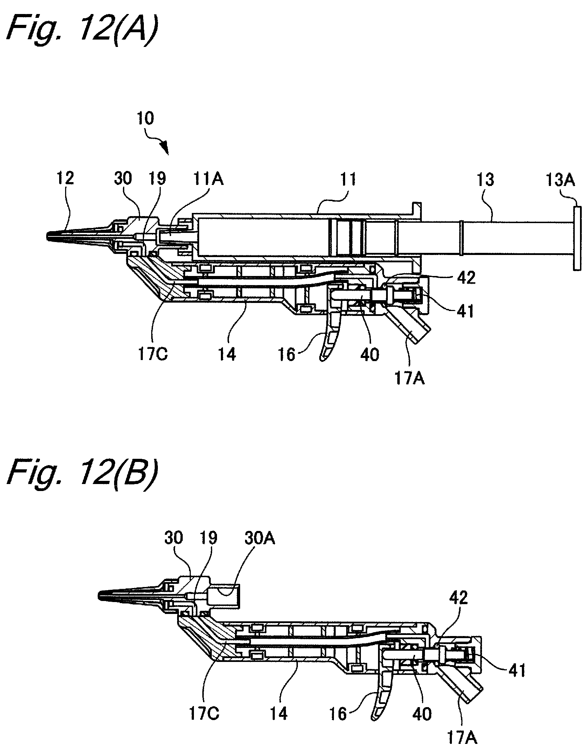

Fig. 12(A) is a cross-sectional view illustrating an applicator according to another embodiment (a fifth embodiment) of the present invention. In comparison with, for example, the applicator 10 illustrated in Figs. 1(A) to 1(E), the applicator 10 illustrated in Fig. 12(A) is generally configured to be unequipped with the grip unit 15 and include the support unit 14 with the trigger (operation unit) 16 disposed thereat, which is instead disposed at this grip unit 15 in the previous embodiment.

More specifically, the applicator 10 according to the present embodiment is configured in such a manner that the push portion 13A of the pusher 13 inserted in the syringe 11 is located at a position that allows the operator to push the push portion 13A with the thumb of the hand engaging the trigger (operation unit) 16 with at least one finger other than this thumb, similarly to the applicators 10 described in the first to fourth embodiments. Therefore, even though being unable to hold the grip unit with his/her middle finger, ring finger, and little finger, the operator can, for example, place his/her thumb on the push portion 13A of the pusher 13 while engaging the trigger 16 with his/her index finger, and easily adjust the injection amount of the liquid agent and/or the injection amount of the compressed air that are injected from the liquid agent injection unit 12 by operating the applicator 10 with this or these index finger and/or thumb.

Further, as illustrated in Fig. 12(B), the applicator 10 is configured in such a manner that the syringe 11 (including the pusher 13) is easily separable or detachable from the applicator 10. This separability allows the syringe 11 (including the pusher 13) to be replaced with a new one, or the syringe (including the pusher 13) and the support unit 14 to be, for example, washed independently of each other.

As illustrated in Fig. 12(B), a syringe attachment unit 30 is provided at the distal end of the support unit 14. The syringe 11 is configured in such a manner that a syringe nozzle 11A formed at the distal end thereof is inserted in an insertion bore 30A of the syringe attachment unit 30 with the syringe 11 laid on the support unit 14 (refer to Fig. 12(B)).

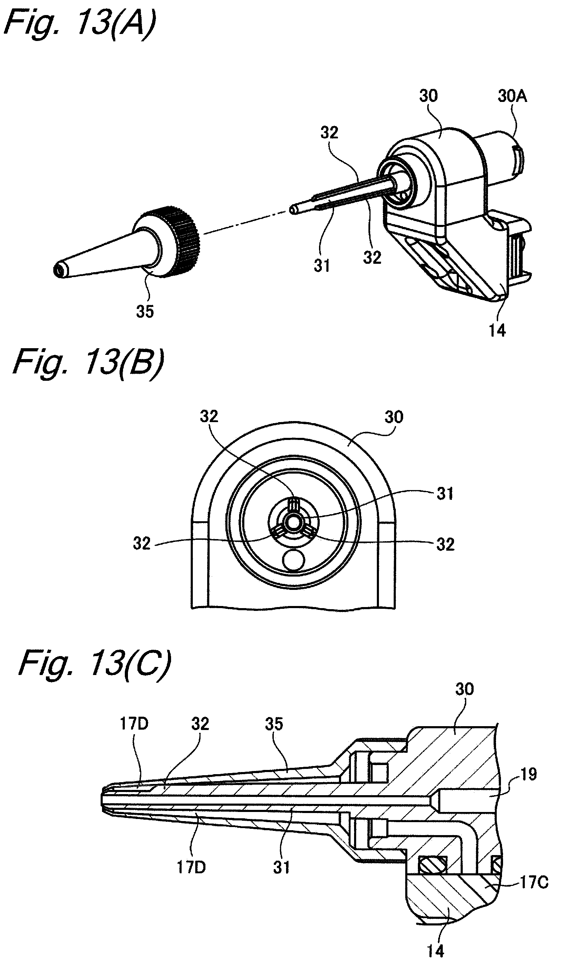

Fig. 13(A) is a perspective view illustrating the syringe attachment unit 30. A nozzle (liquid agent injection unit) 31, which is in communication with the insertion bore 30A, is provided in a protruding manner on an opposite surface of the syringe attachment unit 30 from the insertion bore 30A. The nozzle 31 is arranged so as to be located on a same axis as the syringe 11 attached to the syringe attachment unit 30. The nozzle 31 is configured so as to be in communication with the syringe 11 via a liquid agent flow passage 19 of the syringe attachment unit 30. Further, the nozzle 31 is shaped in such a manner that an inner diameter and an outer diameter thereof are reducing toward a distal end thereof. The nozzle 31 includes a plurality of ribs 32 (for example, three ribs 32 in the drawings) formed on an outer peripheral surface thereof. The plurality of ribs 32 is arranged at equal spaces or even intervals circumferentially, and extends longitudinally. Fig. 13(B) is a front view illustrating the nozzle 31 as viewed from the distal end side, and indicates that the three ribs 32, which protrude radially, are arranged on the outer peripheral surface of the nozzle 31 at intervals of 120°

Then, as illustrated in Fig. 13(A), the syringe attachment unit 30 is configured in such a manner that an air cap 35 is attached to the syringe attachment unit 30 with the nozzle 31 inserted in the air cap 35. The air cap 35 is configured to be able to ensure positional precision between the nozzle 31 and the air cap 35 even with the nozzle 31 deformed due to the ribs 32 formed at the nozzle 31.

As illustrated in Fig. 13(C), an annular air flow passage 17D is defined between the air cap 35 and the outer peripheral surface of the nozzle 31. The air flow passage 17D is in communication with the air flow passage 17C on the support unit 14 side. The respective distal ends of the nozzle 31 and the air cap 35 coincide with each other in axial directions thereof. The applicator 10 is configured in such a manner that the liquid agent from the nozzle 31 and the air from the air flow passage 17D between the nozzle 31 and the air cap 35 are mixed together and injected at the distal ends thereof. The air cap 35 is shaped in such a manner that an inner diameter and an out diameter thereof are reducing toward the distal end thereof.

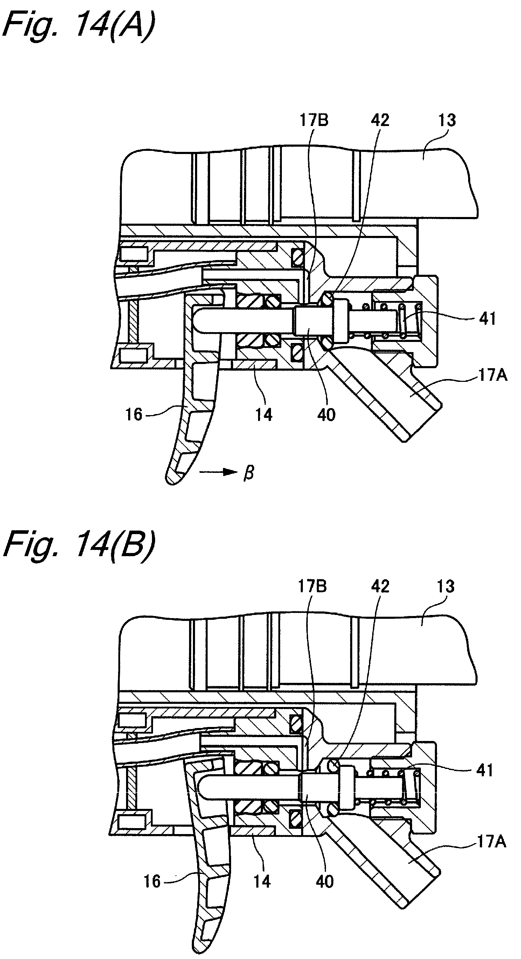

Fig. 14(A) is a cross-sectional view illustrating a configuration in the vicinity of the trigger (operation unit) 16 of the applicator 10 according to the fifth embodiment. The support unit 14 includes, in the vicinity of the end thereof, the air flow passage 17A extending from the gas supply port for supplying the air from outside. The applicator 10 is configured in such a manner that the air from this air flow passage 17A is introduced into the air flow passage 17B and the air flow passage 17C by the operation performed on the trigger (operation unit) 16 in a direction indicated by an arrow β illustrated in the drawing. More specifically, there is provided a shaft member 40 having one end in abutment with the vicinity of a rotational support axis of the trigger (operation unit) 16, and this shaft member 40 is configured to be biased toward the trigger (operation unit) 16 side by a spring 41 disposed on an opposite end of the shaft member 40. An O-ring 42 is mounted at an intermediate portion of the shaft member 40 in a length direction thereof. The O-ring 42 is configured to seal between the air flow passage 17A and the air flow passage 17B, thereby having a function as a valve that cuts off the communication between the air flow passage 17A and the air flow passage 17B, when the trigger (operation unit) 16 is not operated.

Then, as illustrated in Fig. 14(B), the operation performed on the trigger (operation unit) 16 in the direction indicated by the arrow β in the drawing causes the shaft member 40 to be axially displaced against the biasing force of the spring 41, and a displacement of the O-ring 42 according thereto causes the valve to be opened, thereby establishing the communication between the air flow passage 17A and the air flow passage 17B.

### (Sixth Embodiment)

The above-described embodiments have been described assuming that the compressed air is used as the gas for atomizing the liquid agent, by way of example. However, the gas for atomizing the liquid agent is not limited to this example, and it is apparent that carbon dioxide, nitrogen gas, and the like may be used as this gas. In this case, the above-described air flow passage can be caused to function as a gas flow passage, and the above-described air cap can be caused to function as a gas cap.

### (Seventh Embodiment)

The above-described embodiments are embodiments using the hemostatic agent or the coating material as the liquid agent injected from the syringe 11. However, it is apparent that the liquid agent is not limited to these examples, and may be an adhesion prevention agent, a biocompatible adhesive, or the like.

As used herein, the hemostatic agent refers to an agent for use in oozing bleeding and the like, and examples thereof include a fibrin glue. The adhesion prevention agent refers to an agent that is inserted between an organ and another organ or among organs to prevent the organ and the organ from being fixedly attached to each other from close contact therebetween during a surgery and the like. Further, the biocompatible adhesive refers to an adhesive used for the purpose of joining a skin and the like instead of a "suture" during a surgery and the like.

Having described the present invention with use of the embodiments, needless to say, the technical range of the present invention is not limited to the range described in the above-described embodiments. It is apparent to those of skilled in the art that the above-described embodiments can be changed or modified in various manners. Further, it is apparent from the contentns of the claims that the technical range of the present invention can include even the embodiments changed or modified in this manner.

The present application claims priority under the Paris Convention to Japanese Patent Application No. 2013-134120 filed on June 26, 2013, and Japanese Patent Application No. 2014-63290 filed on March 26, 2014. The entire disclosure of each of Japanese Patent Application No. 2013-134120 filed on June 26, 2013, and Japanese Patent Application No. 2014-63290 filed on March 26, 2014 including the specification, the claims, the drawings, and the summary is incorporated herein by reference in its entirety.

The entire disclosure of each of Japanese Patent Application Public Disclosure No. S61-79452 (PTL 1), and Japanese Utility Model Application Public Disclosure No. H6-52839 (PTL 2) including the specification, the claims, the drawings, and the summary is incorporated herein by reference in its entirety.

### REFERENCE SIGNS LIST

- 10: applicator

- 11: syringe
- 11A: syringe nozzle
- 12: liquid agent injection unit
- 12A: nozzle
- 12B: air cap (gas cap)
- 13: pusher
- 13A: push portion
- 14: support unit
- 14E: extending portion
- 14B: back portion
- 141: axial projection body
- 142: projection
- 15: grip unit
- 16: trigger (operation unit)
- 17: air flow passage (gas flow passage)
- 18: valve unit
- 19: liquid agent flow passage
- 20: shaft member
- 21: spring
- 22: button
- 30: syringe attachment unit
- 31: nozzle
- 35: air cap
- 40: shaft member
- 41: spring
- 42: O-ring

## Claims

1. An applicator comprising:
a syringe in which a liquid agent is loaded;
a liquid agent injection unit provided at one end of the syringe and arranged into communication with a liquid agent flow passage;
a pusher configured to be slidably inserted from an opposite end of the syringe;
a push portion provided at an end of the pusher opposite from a syringe side of the pusher that is inserted in the syringe, the push portion being configured to cause the liquid agent loaded in the syringe to be injected from the liquid agent injection unit by being pushed;
a support unit configured to support the syringe;
a gas flow passage formed in the support unit and configured to supply compressed gas to the liquid agent injection unit; and
an operation unit provided to the support unit and configured to cause a start of the supply of the compressed gas to the liquid agent injection unit,
wherein the push portion is located at a position that allows an operator to push the push portion.

2. The applicator according to claim 1, wherein the push portion is located at a position that allows the operator to push the push portion with a thumb of a hand engaging the operation unit with at least one finger other than this thumb.

3. The applicator according to claim 1, wherein the push portion is located at a position that allows the operator to push the push portion with a thumb while holding the operation unit.

4. The applicator according to any one of claims 1 to 3, wherein the support unit can support only a single syringe.

5. The applicator according to any one of claims 1, 2, and 4, wherein the operation unit is provided in such a manner that only the operation unit protrudes from the support unit opposite from the syringe, and the operation unit is not provided with a grip unit.

6. The applicator according to claim 5, further comprising a gas supply port formed in a vicinity of an end of the support unit where the opposite end of the syringe is located, the gas supply port extending obliquely so as to be separating from the syringe,
wherein the compressed gas is supplied from a gas supply hose fitted to the gas supply port.

7. The applicator according to any one of claims 1 to 4, further comprising a grip unit provided to the support unit and including a gas flow passage for supplying the compressed gas to the gas flow passage of the support unit,
wherein the push portion is located at a position that allows the operator to push the push portion while operating the operation unit with a hand holding the grip unit.

8. The applicator according to claim 7, wherein the grip unit is disposed offset from the syringe in a direction intersecting with a longitudinal direction of the syringe.

9. The applicator according to claim 7 or 8, wherein the grip unit is provided to the support unit via an angle adjustment mechanism.

10. The applicator according to any one of claims 1 to 9, wherein the liquid agent injection unit includes a nozzle, and a gas cap provided around the nozzle on an outer periphery of the nozzle, and
wherein the gas cap and the nozzle each are shaped in such a manner that an inner diameter and an outer diameter thereof are reducing toward a distal end thereof.

11. The applicator according to claim 10, wherein the nozzle includes a rib provided on the outer periphery of the nozzle.

12. The applicator according to any one of claims 1 to 11, wherein the syringe is supported by the support unit via a lure-lock mechanism.

13. The applicator according to any one of claims 1 to 12, wherein the pusher causes the liquid agent to be injected form the liquid agent injection unit by pushing the liquid agent into the syringe.

14. The applicator according to any one of claims 1 to 13, wherein the operation unit includes a mechanism capable of adjusting a supply amount of the compressed gas, and atomizes the liquid agent injected form the liquid agent injection unit with use of the compressed gas to spray the atomized liquid agent.

15. The applicator according to any one of claims 1 to 14, wherein the entire applicator is made from resin.

16. The applicator according to any one of claims 1 to 15, wherein the liquid agent is any one of a hemostatic agent, an adhesion prevention agent, and a biocompatible adhesive.
